(19) Europäisches Patentamt / European Patent Office / Office européen des brevets

(11) **EP 3 920 781 B1**

(12) **EUROPEAN PATENT SPECIFICATION**

(45) Date of publication and mention of the grant of the patent:
**22.10.2025 Bulletin 2025/43**

(21) Application number: **20753197.1**

(22) Date of filing: **10.02.2020**

(51) International Patent Classification (IPC):
*A61B 5/00* (2006.01)   *G06N 3/02* (2006.01)
*A61B 5/374* (2021.01)   *G06N 3/04* (2023.01)
*G06N 3/08* (2023.01)   *G06N 7/00* (2023.01)
*G06N 20/00* (2019.01)   *G16H 50/20* (2018.01)

(52) Cooperative Patent Classification (CPC):
**G06N 20/00; A61B 5/374; A61B 5/4094;
A61B 5/7264; G06N 3/045; G06N 3/0464;
G06N 3/08; G06N 3/09; G06N 7/01; G16H 50/20**

(86) International application number:
**PCT/SG2020/050062**

(87) International publication number:
**WO 2020/162837 (13.08.2020 Gazette 2020/33)**

(54) **METHOD AND SYSTEM FOR SEIZURE DETECTION**

VERFAHREN UND SYSTEM ZUR ANFALLSDETEKTION

PROCÉDÉ ET SYSTÈME DE DÉTECTION DE CRISE D'ÉPILEPSIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.02.2019 SG 10201901109U**

(43) Date of publication of application:
**15.12.2021 Bulletin 2021/50**

(73) Proprietor: **Nanyang Technological University
Singapore 639798 (SG)**

(72) Inventors:
  • **DAUWELS, Justin**
    **Singapore 639798 (SG)**
  • **RAJAMANICKAM, Yuvaraj**
    **Singapore 639798 (SG)**

(74) Representative: **Viering, Jentschura & Partner
mbB
Patent- und Rechtsanwälte
Am Brauhaus 8
01099 Dresden (DE)**

(56) References cited:
CN-A- 105 249 962    US-A1- 2013 274 625
US-A1- 2013 274 625    US-A1- 2014 121 554

• YUAN YE ET AL: "A Multi-View Deep Learning Framework for EEG Seizure Detection", IEEE JOURNAL OF BIOMEDICAL AND HEALTH INFORMATICS, IEEE, PISCATAWAY, NJ, USA, vol. 23, no. 1, 1 January 2019 (2019-01-01), pages 83 - 94, XP011695802, ISSN: 2168-2194, [retrieved on 20190103], DOI: 10.1109/JBHI.2018.2871678
• DASH DEBA PRASAD DEBA DASH@THAPAR EDU ET AL: "Review of Machine and Deep Learning Techniques in Epileptic Seizure Detection using Physiological Signals and Sentiment Analysis", ACM TRANSACTIONS ON ASIAN AND LOW-RESOURCE LANGUAGE INFORMATION PROCESSING, ACM, 2 PENN PLAZA, SUITE 701 NEW YORK NY 10121-0701 USA, 1 January 1990 (1990-01-01), XP058733908, ISSN: 2375-4699, DOI: 10.1145/3552512

EP 3 920 781 B1

- WONG S. ET AL.: "A Stochastic Framework for Evaluating Seizure Prediction Algorithms Using Hidden Markov Models. Journal of Neurophysiology", INNOVATIVE METHODOLOGY, vol. 97, no. 3, 4 October 2006 (2006-10-04), pages 2525 - 2532, XP002712913, [retrieved on 20200308]

# EP 3 920 781 B1

**Description**

## CROSS-REFERENCE TO RELATED APPLICATION

This application claims the benefit of priority of Singapore Patent Application No. 10201901109U, filed 8 February 2019.

## TECHNICAL FIELD

The present invention generally relates to a method for seizure detection and a system thereof.

## BACKGROUND

Epilepsy is a neurological condition involving the brain that makes people more susceptible to having recurrent seizures. It is the fourth most frequent disorder of the nervous system and affects people of all ages, races, and ethnic backgrounds. Worldwide, more than 65 million people have this disorder and its exact cause may not be known. The clinical symptoms of epileptic seizures might affect the motor, sensory, memory and cognition of the patient. Epilepsy can be clinically diagnosed by examinations, such as computed tomography (CT), magnetic resonance imaging (MRI), magneto-encephalogram (MEG), positron emission tomography (PET) or electroencephalogram or electroencephalography (EEG). Among these methods, EEG may be preferred due to affordability and efficient temporal resolution. EEG provides direct measurements of the electrical activity in the brain, and has been in clinical use for over 70 years. However, monitoring EEG of a patient over several days is usually required for accurate diagnosis and identification of epileptic seizures. This manual monitoring of EEG is tedious and time consuming. In addition, the result of the interpretation in the same recording can vary from different annotators. Thus, there exists a significant need for automated seizure detection system that would help the long-term practice of EEG monitoring and treatment planning for epilepsy patients.

In recent years, a variety of machine learning and bioengineering-based methods have been applied for seizure detection from scalp EEG. Most of the studies employ manually-designed features that characterize seizure manifestations in EEG; most methods rely on spectral information, whereas some of them employ the temporal characteristics of seizures. However, epileptic seizures are non-stationary in nature with seizure manifestations in EEG being extremely irregular, both within a patient over time and between different patients. Consequently, finding the optimal features for seizure detection is still a challenging as well as important problem.

Neural networks are computational approach inspired by the biological nervous systems. They have been utilized in a wide range of applications including physiological signal analysis of electromyogram, and EEG signals. For example, a deep neural network may comprise several stacked layers of hidden layer neurons. Over the past decade, deep neural learning, a sub-field of machine learning, has achieved remarkable success in various artificial intelligence fields such as computer vision, pattern recognition, and natural language processing. Indeed, features identified by deep learning models have often proven to be more robust than hand-crafted features in various applications. Convolutional Neural Networks (CNNs) are deep learning techniques inspired by the functioning of animal visual cortex. They allow the extraction of higher-level features without human intervention from the original input data, unlike most traditional machine learning algorithms. However, deep neural networks such as CNNs are typically used as a static model whose input feature is fixed-dimensional.

US 2013/274625 A1 describes a neurostimulation device including a plurality of electrodes adapted to be electrically connected to a subject to receive multichannel electrical signals from the subject's brain, a multichannel seizure detection unit electrically connected to the plurality of electrical leads to receive the multichannel electrical signals, and a neurostimulation unit in communication with the multichannel seizure detection unit. The plurality of electrodes are at least three electrodes such that the multichannel electrical signals are at least three channels of electrical signals, and the multichannel seizure detection unit detects a presence of a seizure based on multichannel statistics from the multichannel electrical signals including higher order combinations than two-channel combinations.

A need therefore exists for seizure detection that seek to overcome, or at least ameliorate, one or more of the deficiencies in conventional seizure detection, such as to improve accuracy and/or reliability. It is against this background that the present invention has been developed.

## SUMMARY

According to a first aspect of the present invention, there is provided a computer-implemented method for seizure detection using at least one processor, as defined in claim 1.

According to a second aspect of the present invention, there is provided a system for seizure detection, as defined in claim 8.

According to a third aspect of the present invention, there is provided a computer program product, embodied in one or more non-transitory computer-readable storage mediums, comprising instructions executable by at least one processor to perform a method for seizure detection, as defined in claim 15.

**BRIEF DESCRIPTION OF THE DRAWINGS**

Embodiments of the present invention will be better understood and readily apparent to one of ordinary skill in the art from the following written description, by way of example only, and in conjunction with the drawings, in which:

FIG. 1 depicts a schematic flow diagram of a method for seizure detection using at least one processor according to various embodiments of the present invention;

FIG. 2 depicts a schematic block diagram of a system for seizure detection according to various embodiments of the present invention, such as corresponding to the method shown in FIG. 1;

FIG. 3 depicts an example computer system which the system for seizure detection according to various embodiments of the present invention may be embodied in;

FIG. 4 illustrates a diagram of an exemplary seizure detection framework for detecting seizure in a brain signal data according to various example embodiments of the present invention;

FIG. 5A illustrates an exemplary architecture of a convolutional neural network (CNN) which may be used for detecting seizure in the brain signal data according to various example embodiments of the present invention;

FIG. 5B illustrates an exemplary graph of a Hidden Markov Model (HMM);

FIG. 5C illustrates an exemplary graph of a Conditional Random Field (CRF) model;

FIG. 6 illustrates an exemplary diagram of brain signal data, CNN classification output data (first processed output data) and post-processing data of the CNN classification output data by statistical models (second processed output data);

FIG. 7A shows an exemplary graph illustrating sensitivity with respect to time margin of seizure detection using a CNN and a Conditional Random Field (CRF) statistical model;

FIG. 7B shows an exemplary box plot for detection latencies;

FIG. 8 shows an exemplary graph illustrating the sensitivity vs. the false detection rate (FDR) of the seizure detection framework according to various embodiments of the present invention;

FIGS. 9A-9B illustrate exemplary spectrograms for seizure EEG segments and non-seizure EEG segments, respectively; and

FIGS. 10A-10B illustrate exemplary scalograms for seizure EEG segments and non-seizure EEG segments, respectively.

**DETAILED DESCRIPTION**

Various embodiments of the present invention provide a method (computer-implemented method) and a system (including a memory and at least one processor communicatively coupled to the memory) for seizure detection. In various embodiments, a hybrid model for automatic seizure detection from brain signal data using a deep neural network (or deep learning model) and a statistical model is provided. The deep neural network may be facilitated with a dynamic temporal model to enable the deep neural network to be applied to a dynamic signal sequence problem. In accordance with one aspect, the statistical model may be used for post-processing output data from the deep neural network. The statistical model may be configured to model transitions between seizure events and non-seizure events of the output data of the deep neural network, capturing the dynamics of the sequential data (output data of the deep neural network). Accordingly, a determination of one or more seizure events in the brain signal data may be made based on the processed output data obtained using the deep neural network and the statistical model.

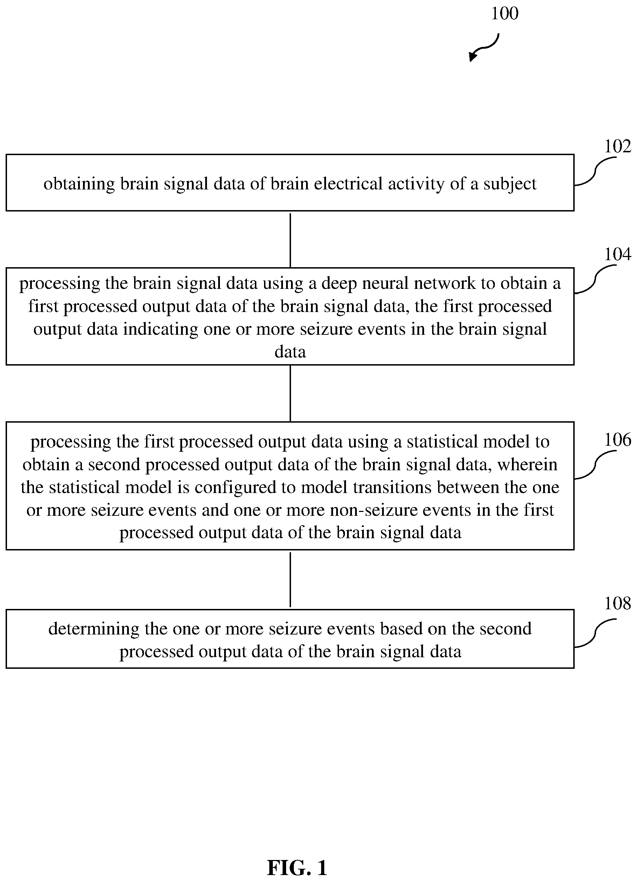

FIG. 1 depicts a schematic flow diagram of a method 100 (computer-implemented method) for seizure detection using at least one processor according to various embodiments of the present invention. The method 100 comprises obtaining (at 102) brain signal data of brain electrical activity of a subject; processing (at 104) the brain signal data using a deep neural network or deep learning model to obtain a first processed output data of the brain signal data (or first processed brain signal data), the first processed output data indicating one or more seizure events in the brain signal data; processing (at 106) the first processed output data using a statistical model to obtain a second processed output data of the brain signal data (or second processed brain signal data), wherein the statistical model is configured to model transitions between the one or more seizure events and one or more non-seizure events in the first processed output data of the brain signal data; and determining (at 108), the one or more seizure events based on the second processed output data of the brain signal data.

In relation to 102, in various embodiments, the brain signal data comprises electroencephalogram (EEG) signal data of the subject. For example, the brain signal data may be an EEG signal dataset or recordings captured or acquired using an EEG device. Electroencephalography is an electrophysiological monitoring method to record electrical activity of the brain, which is typically non-invasive, with electrodes placed at various positions along the scalp of the subject to measure voltage fluctuations resulting from ionic current within the neurons of the brain. The brain signal data may be a multi-channel brain signal data. It will be appreciated by a person skilled in the art that the present invention is not limited to EEG signal dataset captured using an EEG device and that other electrophysiological signal acquisition techniques known in the art may also be used to obtain brain signal data relating to brain electrical activity of the subject.

In various embodiments, the brain signal data processed using the deep neural network comprises spectral, temporal and spatial information in relation to the one or more seizure events. In various embodiments, an image-based representation of the brain signal data in the time-frequency domain may be further produced, wherein the above-mentioned processing the brain signal data using a deep neural network comprises processing the image-based representation to obtain the first processed output data. The image-based representation may represent the brain signal data in both the time and frequency domains simultaneously. The image-based representation may enable spectral, temporal and spatial information existing in the one or more seizure events to be captured simultaneously. For example, time-frequency images demonstrate the joint distribution information of time domain and frequency domain of the original brain signal, which may provide abundant information for seizure detection.

In various embodiments, the image-based representation may be a spectrogram. The spectrogram may be a visual representation of the brain signal in the time-frequency domain using the short time Fourier transform (STFT).

In various embodiments, the image-based representation may be a scalogram. The scalogram may be a visual representation of the brain signal in the time-frequency domain using the wavelet transform (WT).

In various embodiments, the brain signal data comprises a plurality of signals, each of the plurality of signals corresponding to a respective channel that is associated with a different brain spatial location (or electrode position). The brain signal data may be arranged as a three-dimensional (3D) tensor, where the three dimensions are space (e.g., obtained by each channel corresponding to a respective electrode positioned on the scalp), time and frequency, and fed into the deep neural network. In various embodiments, the deep neural network (or deep learning model) may be a convolutional neural network (CNN). In various embodiments, the above-mentioned processing the brain signal data using a deep neural network further comprises convolving each of the plurality of signals with a one-dimensional linear finite impulse response filter.

The first processed output data of the brain signal data may comprise a first time-series signal indicating one or more seizure events in the brain signal data. For example, the first time-series signal may indicate the presence of one or more seizure events over time. In various embodiments, the first processed output data of the brain signal data may be a classification output data. The first processed output data, for example, may comprise quantitative values within a predetermined range. In various embodiments, the quantitative values may be probability values between the predetermined range, such as from the range of 0 to 1 in a non-limiting example, with higher values representing a seizure waveform.

The deep neural network may be trained using a brain signal dataset comprising brain signal segments relating to seizure events and brain signal segments relating to non-seizure events.

In relation to 106, the statistical model may be configured to determine whether a segment or portion of the first processed output data belongs to a seizure state or a non-seizure state. In various embodiments, the statistical model may be configured to generate probability values indicating a seizure state or a non-seizure state. For example, a probability value (or statistical output) 0 indicating a non-seizure state and/or a probability value (or statistical output) 1 indicating a seizure state may be generated. The second processed output data of the brain signal data may comprise a second time-series signal indicating one or more seizure events in the brain signal data. For example, the second time-series signal may indicate the presence of one or more seizure events over time.

In various embodiments, the first processed output data may comprise outliers or fluctuations. In various embodiments, the above-mentioned processing the first processed output data using a statistical model to obtain a second processed output data of the brain signal data further comprises reducing or removing the outliers in the first processed output data by the statistical model. In various embodiments, the statistical model may be configured to refine the first processed output data (e.g., refine the time-series signal).

In various embodiments, the statistical model may comprise a Hidden Markov Model (HMM). In various embodiments, the statistical model may comprise a Conditional Random Field (CRF).

In various embodiments, the brain signal data may be segmented into a plurality of different spectral bands. For example, different combinations of the spectral bands may be fed into the CNN, either concatenated into one long vector, or arranged as a matrix.

The automatic seizure detection may advantageously reduce the time to diagnosis and enhance real-time applications such as ICU monitoring.

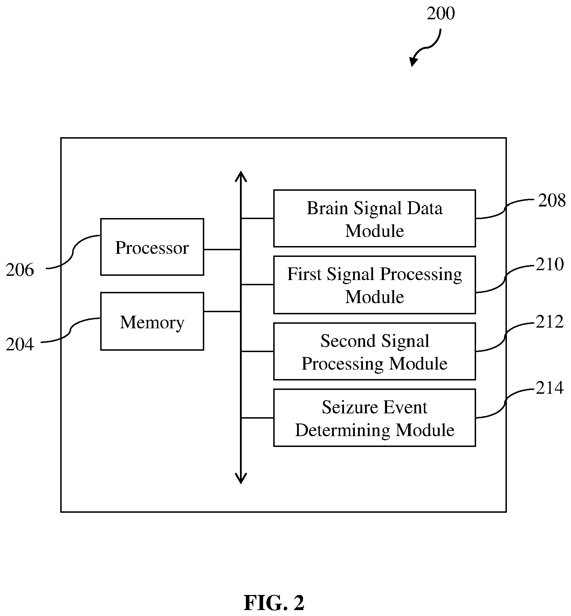

FIG. 2 depicts a schematic block diagram of a system 200 for seizure detection according to various embodiments of the present invention, such as corresponding to the method 100 for seizure detection as described hereinbefore according to various embodiments of the present invention.

The system 200 comprises a memory 204, and at least one processor 206 communicatively coupled to the memory 204 and configured to: obtain brain signal data of brain electrical activity of a subject; process the brain signal data using a deep neural network to obtain a first processed output data of the brain signal data, the first processed output data indicating one or more seizure events in the brain signal data; process the first processed output data using a statistical model to obtain a second processed output data of the brain signal data, wherein the statistical model is configured to model transitions between the one or more seizure events and one or more non-seizure events in the first processed output data of the brain signal data; and determine the one or more seizure events based on the second processed output data of the brain signal data.

It will be appreciated by a person skilled in the art that the at least one processor 206 may be configured to perform the required functions or operations through set(s) of instructions (e.g., software modules) executable by the at least one processor 206 to perform the required functions or operations. Accordingly, as shown in FIG. 2, the system 200 may further comprise a brain signal data module (or circuit) 208 configured to obtain brain signal data of brain electrical activity of a subject; a first signal processing module (or circuit) 210 configured to process the brain signal data using a deep neural network to obtain a first processed output data of the brain signal data; a second signal processing module (or circuit) 212 configured to process the first processed output data using a statistical model to obtain a second processed output data of the brain signal data; and a seizure event determining module (or circuit) 214 configured to determine one or more seizure events based on the second processed output data of the brain signal data.

It will be appreciated by a person skilled in the art that the above-mentioned modules (or circuits) are not necessarily separate modules, and two or more modules may be realized by or implemented as one functional module (e.g., a circuit or a software program) as desired or as appropriate without deviating from the scope of the present invention. For example, the brain signal data module 208, the first signal processing module 210, the second signal processing module 212, and/or the seizure event determining module 214 may be realized (e.g., compiled together) as one executable software program (e.g., software application or simply referred to as an "app"), which for example may be stored in the memory 204 and executable by the at least one processor 206 to perform the functions/operations as described herein according to various embodiments.

In various embodiments, the system 200 corresponds to the method 100 as described hereinbefore with reference to FIG. 1, therefore, various functions/operations configured to be performed by the least one processor 206 may correspond to various steps or operations of the method 100 described hereinbefore according to various embodiments, and thus need not be repeated with respect to the system 200 for clarity and conciseness. In other words, various embodiments described herein in context of the methods are analogously valid for the respective systems (e.g., which may also be embodied as devices).

For example, in various embodiments, the memory 204 may have stored therein the brain signal data module 208, the first signal processing module 210, the second signal processing module 212, and/or the seizure event determining module 214, which respectively correspond to various steps or operations of the method 100 as described hereinbefore, which are executable by the at least one processor 206 to perform the corresponding functions/operations as described herein.

A computing system, a controller, a microcontroller or any other system providing a processing capability may be provided according to various embodiments in the present disclosure. Such a system may be taken to include one or more processors and one or more computer-readable storage mediums. For example, the system 200 described hereinbefore may include a processor (or controller) 206 and a computer-readable storage medium (or memory) 204 which are for example used in various processing carried out therein as described herein. A memory or computer-readable storage medium used in various embodiments may be a volatile memory, for example a DRAM (Dynamic Random Access Memory) or a non-volatile memory, for example a PROM (Programmable Read Only Memory), an EPROM (Erasable PROM), EEPROM (Electrically Erasable PROM), or a flash memory, e.g., a floating gate memory, a charge trapping memory, an MRAM (Magnetoresistive Random Access Memory) or a PCRAM (Phase Change Random Access Memory).

In various embodiments, a "circuit" may be understood as any kind of a logic implementing entity, which may be special purpose circuitry or a processor executing software stored in a memory, firmware, or any combination thereof. Thus, in an embodiment, a "circuit" may be a hard-wired logic circuit or a programmable logic circuit such as a programmable processor, e.g., a microprocessor (e.g., a Complex Instruction Set Computer (CISC) processor or a Reduced Instruction Set Computer (RISC) processor). A "circuit" may also be a processor executing software, e.g., any kind of computer program, e.g., a computer program using a virtual machine code, e.g., Java. Any other kind of implementation of the respective functions which will be described in more detail below may also be understood as a "circuit" in accordance with various alternative embodiments. Similarly, a "module" may be a portion of a system according to various embodiments in the present invention and may encompass a "circuit" as above, or may be understood to be any kind of a logic-implementing entity therefrom.

Some portions of the present disclosure are explicitly or implicitly presented in terms of algorithms and functional or symbolic representations of operations on data within a computer memory. These algorithmic descriptions and functional or symbolic representations are the means used by those skilled in the data processing arts to convey most effectively the substance of their work to others skilled in the art. An algorithm is here, and generally, conceived to be a self-consistent sequence of steps leading to a desired result. The steps are those requiring physical manipulations of physical quantities, such as electrical, magnetic or optical signals capable of being stored, transferred, combined, compared, and otherwise manipulated.

Unless specifically stated otherwise, and as apparent from the following, it will be appreciated that throughout the present specification, discussions utilizing terms such as "determining", "obtaining", "processing", "producing", or the like, refer to the actions and processes of a computer system, or similar electronic device, that manipulates and transforms data represented as physical quantities within the computer system into other data similarly represented as physical quantities within the computer system or other information storage, transmission or display devices.

The present specification also discloses a system (which may also be embodied as a device or an apparatus) for performing the operations/functions of the methods described herein. Such a system may be specially constructed for the required purposes, or may comprise a general purpose computer or other device selectively activated or reconfigured by a computer program stored in the computer. The algorithms presented herein are not inherently related to any particular computer or other apparatus. Various general-purpose machines may be used with computer programs in accordance with the teachings herein. Alternatively, the construction of more specialized apparatus to perform the required method steps may be appropriate.

In addition, the present specification also at least implicitly discloses a computer program or software/functional module, in that it would be apparent to the person skilled in the art that the individual steps or operations of the methods described herein may be put into effect by computer code. The computer program is not intended to be limited to any particular programming language and implementation thereof. It will be appreciated that a variety of programming languages and coding thereof may be used to implement the teachings of the disclosure contained herein. Moreover, the computer program is not intended to be limited to any particular control flow. There are many other variants of the computer program, which can use different control flows without departing from the scope of the invention. It will be appreciated by a person skilled in the art that various modules described herein (e.g., the brain signal data module 208, the first signal processing module 210, the second signal processing module 212, and/or the seizure event determining module 214) may be software module(s) realized by computer program(s) or set(s) of instructions executable by a computer processor to perform the required functions, or may be hardware module(s) being functional hardware unit(s) designed to perform the required functions. It will also be appreciated that a combination of hardware and software modules may be implemented.

Furthermore, one or more of the steps or operations of a computer program/module or method described herein may be performed in parallel rather than sequentially. Such a computer program may be stored on any computer readable medium. The computer readable medium may include storage devices such as magnetic or optical disks, memory chips, or other storage devices suitable for interfacing with a general-purpose computer. The computer program when loaded and executed on such a general-purpose computer effectively results in an apparatus that implements the steps or operations of the methods described herein.

In various embodiments, there is provided a computer program product, embodied in one or more computer-readable storage mediums (non-transitory computer-readable storage medium), comprising instructions (e.g., the brain signal data module 208, the first signal processing module 210, the second signal processing module 212, and/or the seizure event determining module 214) executable by one or more computer processors to perform a method 100 for seizure detection as described hereinbefore with reference to FIG. 1. Accordingly, various computer programs or modules described herein may be stored in a computer program product receivable by a system (e.g., a computer system or an electronic device) therein, such as the system 200 as shown in FIG. 2, for execution by at least one processor 206 of the system 200 to perform the required or desired functions.

The software or functional modules described herein may also be implemented as hardware modules. More particularly, in the hardware sense, a module is a functional hardware unit designed for use with other components or modules. For example, a module may be implemented using discrete electronic components, or it can form a portion of an entire electronic circuit such as an Application Specific Integrated Circuit (ASIC). Numerous other possibilities exist. Those skilled in the art will appreciate that the software or functional module(s) described herein can also be implemented as a combination of hardware and software modules.

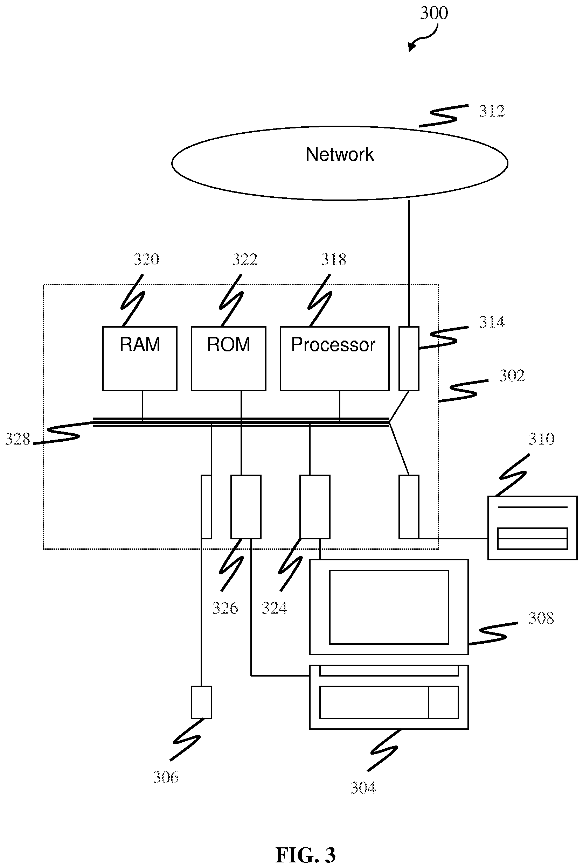

In various embodiments, the above-mentioned computer system may be realized by any computer system (e.g., portable or desktop computer system), such as a computer system 300 as schematically shown in FIG. 3 as an example only and without limitation. Various methods/operations or functional modules (e.g., the brain signal data module 208, the first signal processing module 210, the second signal processing module 212, and/or the seizure event determining module 214) may be implemented as software, such as a computer program being executed within the computer system 300, and instructing the computer system 300 (in particular, one or more processors therein) to conduct the methods/-functions of various embodiments described herein. The computer system 300 may comprise a computer module 302, input modules, such as a keyboard 304 and a mouse 306, and a plurality of output devices such as a display 308, and a printer 310. The computer module 302 may be connected to a computer network 312 via a suitable transceiver device 314, to enable access to e.g. the Internet or other network systems such as Local Area Network (LAN) or Wide Area Network (WAN). The computer module 302 in the example may include a processor 318 for executing various instructions, a Random Access Memory (RAM) 320 and a Read Only Memory (ROM) 322. The computer module 302 may also include a number of Input/Output (I/O) interfaces, for example I/O interface 324 to the display 308, and I/O interface 326 to the keyboard 304. The components of the computer module 302 typically communicate via an interconnected bus 328 and in a manner known to the person skilled in the relevant art.

It will be appreciated by a person skilled in the art that the terminology used herein is for the purpose of describing various embodiments only and is not intended to be limiting of the present invention. As used herein, the singular forms "a", "an" and "the" are intended to include the plural forms as well, unless the context clearly indicates otherwise. It will be further understood that the terms "comprises" and/or "comprising", or the like such as "includes" and/or "including", when used in this specification, specify the presence of stated features, integers, steps, operations, elements, and/or components, but do not preclude the presence or addition of one or more other features, integers, steps, operations, elements, components, and/or groups thereof.

In order that the present invention may be readily understood and put into practical effect, various example embodiments of the present invention will be described hereinafter by way of examples only and not limitations. It will be appreciated by a person skilled in the art that the present invention may, however, be embodied in various different forms or configurations and should not be construed as limited to the example embodiments set forth hereinafter. Rather, these example embodiments are provided so that this disclosure will be thorough and complete, and will fully convey the scope of the present invention to those skilled in the art.

In particular, for better understanding of the present invention and without limitation or loss of generality, various example embodiments of the present invention will now be described with respect to seizure detection in scalp EEG signal data of a subject, such as an epileptic patient. The seizure detection in the EEG signal data may be described with respect to epileptic seizure of an epileptic patient, however, it will be appreciated by a person skilled in the art that the seizure may be related to and caused by other known neurological medical conditions.

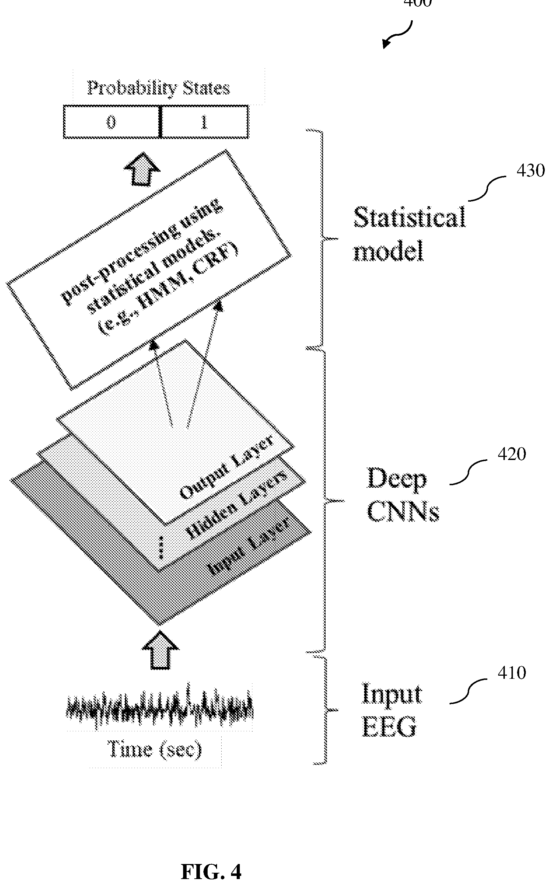

In various example embodiments, the EEG signal of the subject may be recorded using an EEG device comprising a plurality of scalp electrodes (each electrode corresponding to a respective channel), an amplifier and processor. FIG. 4 illustrates a diagram of an exemplary seizure detection framework 400 for detecting seizure in the brain signal data according to various example embodiments of the present invention. In various example embodiments, the seizure detection framework 400 comprises a deep neural network 420 and a statistical model 430. The EEG signal data 410 may be processed using the deep neural network 420, such as a deep CNN, to obtain a first processed output data of the EEG signal data.

The deep neural network 420 may be trained using a brain signal dataset comprising brain signal segments relating to seizure events and brain signal segments relating to non-seizure events. In various example embodiments, the deep neural network 420 may be trained using an EEG signal dataset comprising seizure events in a plurality of subjects obtained from a database such as the publicly available Children's Hospital Boston (CHB)-Massachusetts Institute of Technology (MIT) database. The EEG signals of the dataset may be recorded, for example, with a sampling frequency of 256 Hz. The EEG signal dataset may comprise scalp EEG signals recorded from 23 pediatric patients (5 males, 17 females and 1 missing gender information) with 198 annotated epileptic intractable seizures (not controlled with seizure medications). In an example implementation, 983 hours duration of raw EEG recordings from 23 channels were recorded according to the International 10-20 electrodes positioning system. The recordings may be organized in 24 cases, each case comprising EEG signal data from a single subject, except cases 1 and 21 which were obtained from the same subject with a 1.5-year interval. The EEG signal data may be pre-processed. For example, the EEG signal data may be pre-processed to remove recordings with different montages. In an example, it was noted that seventeen seizures files had different montages (monopolar) to the rest of the seizure files (bipolar recordings). Thus, the seventeen recordings were excluded. The EEG records that contained dashes (missing data) and extreme artifacts were also excluded. In the final dataset, EEG recordings from 24 cases with length up to 526 hours' duration comprising 181 seizure events were evaluated. The seizure detection framework 400, for example, may be developed across a group of patients, rather than using data from individual patients i.e., patient specific detector.

The EEG signal data 410 may be pre-processed prior to input into the deep neural network 420. In various example embodiments, the EEG signal data 410 may be pre-processed with a bandpass filter. For example, the scalp EEG data may be pre-processed by applying a 4th order zero phase Butterworth bandpass filter between 0.5 and 30 Hz. In various example embodiments, the EEG data may be segmented into five-second waveforms for CNN learning. For example, there may be 1280 samples for each waveform in a single channel at a sampling rate of 256Hz. In an example, the segmentation resulted in 2141 seizure segments extracted from 181 seizure events. For a balanced training of the CNN, 2141 non-seizure segments may be extracted from the dataset randomly.

In various example embodiments, a four-fold cross-validation may be applied for evaluating the seizure detection framework 400. During the CNN evaluation process, the 24 epileptic cases were divided into four folds, randomly, by keeping the distribution of seizure segments similar in all the folds. Table 1 shows exemplary details of the different fold-split as follows:

Table 1: Details of division of fold

| Fold No. | Case IDs | No of seizure events | No of seizure segments |
|---|---|---|---|
| 1 | CHB-01, 03, 04, 05, 10, 23 | 37 | 518 |
| 2 | CHB-02, 08, 12, 20, 22, 24 | 62 | 594 |
| 3 | CHB-07, 09, 11, 13, 17, 18 | 29 | 482 |
| 4 | CHB-06, 14, 15, 16, 19, 21 | 53 | 547 |
| Total | | 181 | 2141 |

For each cross-validation step, three folds (18 cases) were applied for training and the testing was performed on the remaining fold (6 cases). The CNN model training was terminated once the validation error stopped improving. For example, the cross entropy may be considered as the error function for the training and validation of the CNN architecture. The different hyper-parameters and the stopping criteria of the model were optimized by applying a nested cross-validation on the training dataset: 70% for training and 30% for validation.

In various example embodiments, in order to simultaneously capture spectral, temporal and spatial information existing in a seizure, an image-based representation of the raw EEG data may be used. Time-frequency images demonstrates the joint distribution information of time domain and frequency domain of the original signal, which can provide abundant information for seizure detection. Time frequency represents a signal in both the time and frequency domains simultaneously. In various example embodiments, the time-frequency representations may comprise spectrograms, scalograms, for example. For example, the EEG signal data 410 may be pre-processed using bandpass filtering and spectrogram/scalogram generation, which uses STFT/WT changing the signal into time-frequency domain. These representations may be graphically represented as an image. Spectrograms may be a visual representation of the Short-Time Fourier Transform (STFT) where the $x$- and $y$-axis are time and frequency, respectively, and the intensity (or color scale) of the image indicates the amplitude of the frequency. The basis for the STFT representation is a series of sinusoids. For example, STFT may be the most straightforward frequency domain analysis. However, it cannot adequately model time variant and transient signal. Spectrograms add time to the analysis of FFT allowing the localization of both time and frequency. Scalograms are a graphical image of the wavelet transform (WT). WTs are a linear time-frequency representation with a wavelet basis instead of sinusoidal functions. Due to the addition of a scale variable along with the time variable, the WT may be effective for non-stationary and transient signals.

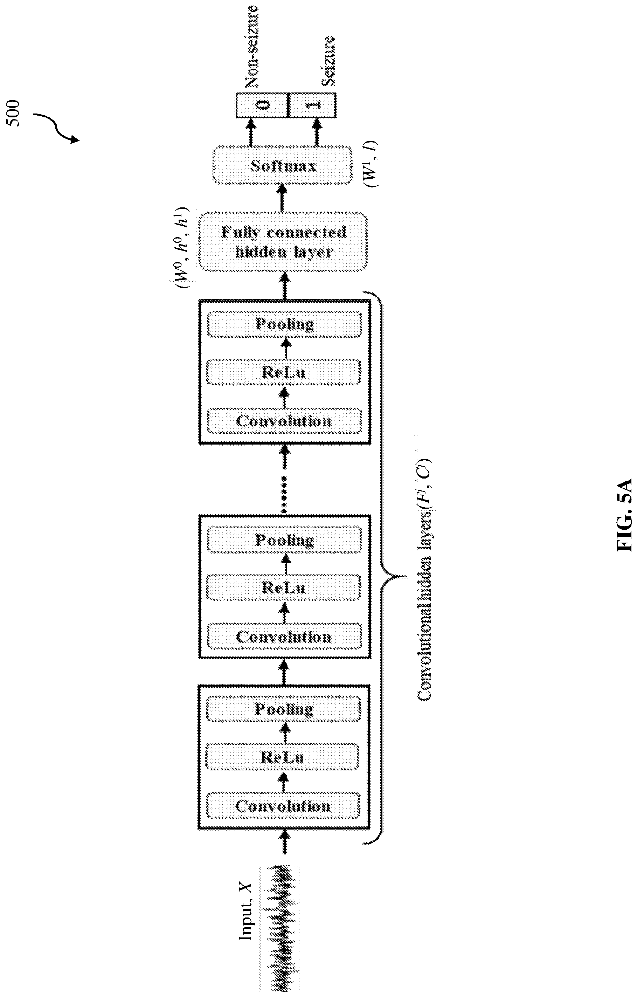

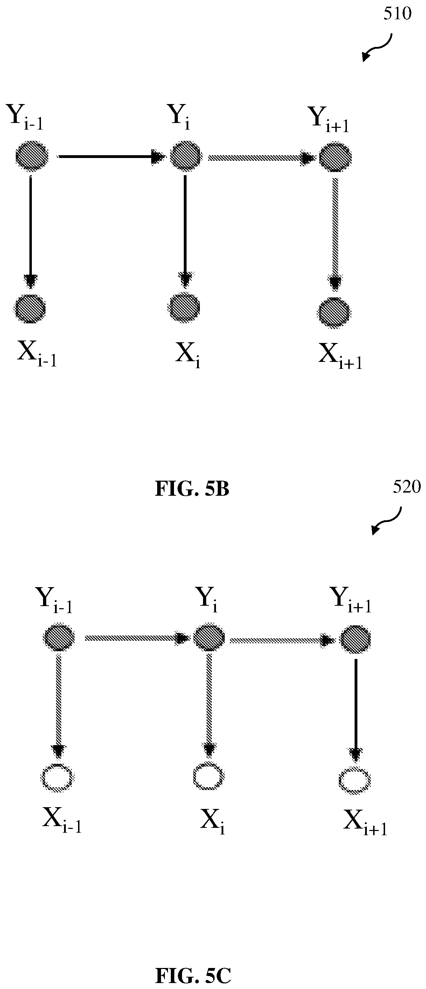

The pre-processed data may be arranged as a three-dimensional (3D) tensor, where the three dimensions may be space (electrodes), time and frequency. This tensor is fed to the CNN with optimal hyper-parameter setting. In various example embodiments, the architecture of the CNN may include an input layer and an output layer, as well as multiple hidden layers made of convolutional layers (applying a convolution operation to the input and passing the result to the next layer), activation functions (defining the output of certain node given an input of set of inputs), pooling layers (combining the outputs of neuron clusters at one layer into a single neuron in the next layer), a fully connected layer at the output (connecting every neuron in one layer to every neuron in another layer). In various example embodiments, some or all of those types of components may be included. FIG. 5 illustrates an exemplary architecture of a CNN 500 which may be used for detecting seizure in the brain signal data according to various example embodiments of the present invention. For example, $X$ denotes the time-series EEG signal as input (1280 time samples $\times$ 1 channel), $F^j$ are the filters for each hidden layer, $C^j$ are the feature maps for each hidden layer, $W^0$ denotes the weight matrix for the fully connected hidden layer, $h^0$ and $h^1$ are the pre-activation and post-activation features for the fully connected hidden layer, $W^1$ and $l$ are the weight matrix and activation of a logistic regressor.

In various example embodiments, the input vector for the CNN may comprise 1280 samples from each channel (dimensions: 1 channel $\times$ 1280). Since seizures often may be observed on one or more channels and may spread to other channels (i.e., seizures spreading spatially), it may be beneficial to combine information from multiple channels in order to detect seizures. Instead of feeding/analyzing each channel separately, the channels may be processed together/jointly by feeding them all into the CNN, which may help to improve the detector performance. Such approach may be referred to as early fusion. By considering multiple channel simultaneously, the CNN learns more information about seizure spatial pattern. In such exemplary case, the input vector for the CNN may be 23 channels $\times$ 1280 samples. Alternatively, signal data from each channel may be processed using the CNN separately to produce output data for each channel respectively. In other words, the CNN may be applied to each channel separately, and the CNN outputs across different channels may be combined (e.g., to obtain the first processed output data). Such approach may be referred to as late fusion. The CNN output of each channel (e.g., 23 seizure detector outputs) may be combined to produce a single time-instant level output. For example, the maximum CNN output (maximum rule) across the channels may be computed, and that value is considered the CNN output (the first processed output data) for that time instant.

According to various example embodiments, a convolution operation may be performed by convolving each electrode signal of the EEG data with a one-dimensional linear finite impulse response filter (to capture the temporal information of the EEG signal) and passed through the output function to form the feature map. In various example embodiments, the output function may be selected as rectifier linear unit (ReLU) function. Following this, a pooling layer may be utilized to provide a squashing function. In various example embodiments, max-pooling may be applied for the pooling layer. The generated features from the convolutional layers are passed through a fully connected hidden layer. In various example embodiments, a logistic regressor may be used to perform classification to seizure and non-seizure classes. In various example embodiments, the logistic regressor may be a softmax function. For example, the CNN output layer may be mapped between [0, 1] using the softmax function. The optimized CNN model may be tested on the testing fold at 5-sec window (1280 samples × 1 channel) with a 75% overlap. For each 5-sec EEG input, the CNN may generate a probability value between [0, 1] with the higher value representing the seizure waveform. In order to obtain the time instant detection, the 21-channel CNN outputs from a single time instant may be combined together. The maximum value of 21-channel may be chosen as the combined time instant output, resulting in a single value, from a range of 0 to 1 in a non-limiting example, for each 5-sec EEG window. These procedures may be repeated until all the folds were employed as test data.

Once a test set is classified using CNN, it is noted that false positives (FPs), i.e., true non-seizure detected as seizure, and false negatives (FNs), i.e., true seizure detected as non-seizure, tend to be sporadic and isolated in time as compared to true positives (TPs), i.e., the number of seizure segments detected correctly, and true negatives (TNs), i.e., the number of true non-seizure segments detected correctly.

The tested CNN output data (the first processed output data) may be processed using the statistical model 430. In various example embodiments, post-processing of the CNN outputs using the statistical model 430 may comprise modelling transitions between classes of the CNN output data. In other words, the statistical model may model transitions between one or more seizure events and one or more non-seizure events in the CNN output data (first processed output data). For example, the statistical model 430 may be used to post-process the CNN outputs so as to accurately capture seizure and non-seizure stage switching. Accordingly, the seizure detection framework 400 comprising the deep neural network 420 and the statistical model 430 may advantageously capture the dynamics of the sequential data (CNN outputs). Additionally, the post-processing of the CNN outputs using the statistical model 430 may be configured to reduce or eliminate undesired outliers or fluctuations of the CNN outputs (the first processed output data). The statistical model 430 may encode the dynamics of seizure EEG; specifically, it encapsulates the fact that usually, seizures last tens of seconds up to several minutes. To learn to capture those dynamics, the statistical model 430 may be trained based on the training fold CNN outputs, and the trained model may be tested by applying it to observations (e.g., CNN outputs) in the test fold.

In various example embodiments, the statistical model 430 may comprise a Hidden Markov Model (HMM). A HMM is a statistical Markov model in which the system state is not directly visible (i.e., hidden). The hidden state may be binary: (i) seizure or (ii) non-seizure. The model may be represented by a set of parameters $\lambda = \{\pi, X, Y\}$, where $\pi$ denotes the initial state probabilities, X denotes the state transition matrix, Y denotes the observation probabilities (e.g., discrete observation probabilities). The probability of the current state of the model $P(S_t \mid Model)$ may be as follows:

$$P\left(S_t \mid Model\right) = P\left(S_t \mid S_{t-1}\right) \cdot P\left(O_t \mid S_t\right) \qquad (1)$$

where $P(S_t \mid S_{t-1})$ denotes the transition probability of a previous state to the current state, $P(O_t \mid S_t)$ denotes the probability of detecting the current observation given the current state, $S_t$ denotes the state at time t, and [.] operator denotes the multiplication. In various example embodiments, the state that maximizes the product of the probabilities may be selected as the state as follows:

$$S_t = arg\ max\ P(S_t \mid Model) \qquad (2)$$

In various example embodiments, the initial estimates of the HMM parameters may be obtained by clustering the CNN output by a Gaussian mixture with two components. The means and covariance matrices of each Gaussian component may be choosen as initial estimates of the HMM parameters of the seizure state and non-seizure state. In various example embodiments, the HMM may be trained on the training fold by the Baum-Welch algorithm to determine the maximum likelihood estimation of the HMMs parameters that converges to a local optimum from an initial guess of the parameters value. Once the HMM model has been trained on the training fold, the observations in the test fold may be decoded by the Viterbi algorithm.

In various example embodiments, the statistical model 430 may comprise a Conditional Random Field (CRF) model. The CRF may be a discriminative graphical model which may capture (e.g., directly) the probabilities of possible label sequences given an observation sequence (e.g., corresponding to CNN outputs), without making independent assumptions on the observation elements. For the modelling of temporal information, it may be sufficient to consider a simple chain (e.g., linear-chain CRF (LCRF)). In various example embodiments, nodes may be fully connected as a bidirectional chain (e.g., each node has two neighbor nodes). In various example embodiments, the observation sequence corresponding to CNN outputs may be denoted as X = {$x_1$, $x_2$, $x_3$ ....,$x_m$}, and the sequence of seizure/non-seizure labels may be denoted as Y = {$y_1$ ,$y_2$, $y_3$ ....,$y_m$}. In various example embodiments, the CRF may be defined as follows:

$$p(Y|X) \propto exp\{\sum_{i=1}^{n} \big[ \sum_{j=1}^{k_1} \lambda_j f_j( y_i - 1, y_i, X, i) + \sum_{j=1}^{k_2} \mu_j g_j(y_i, X, i) \big] \} \qquad (3)$$

where $f_j$ and $g_j$ are Gaussian distributions with means 0 (non-seizure) and 1 (seizure) respectively, and $\lambda_j$ and $\mu_j$ are fitting parameters. In various example embodiments, the parameters in the LCRF may be inferred by maximum likelihood estimation.

FIG. 5B illustrates an exemplary graph 510 of a HMM, while FIG. 5C illustrates an exemplary graph 520 of a CRF model. The graphs may comprise nodes denoted by Y and X. As illustrated, each node $Y_i$ may have two neighbor nodes, $Y_{i-1}$ and $Y_{i+1}$. In various example embodiments, a HMM may have a directed graph. The model may comprise both observed and latent variables. In various example embodiments, a CRF may have an undirected graph and may generate latent variables. An open circle in the graph 520 of the CRF model may indicate that the variable is not generated by the model.

In various example embodiments, for each 5-second CNN output, the statistical model may be configured to generate an observation value (probability value) in a predetermined range, such as in the range from 0 to 1 (e.g., 0, 0.3, 0.8, 1) in a non-limiting example. For example, if the observation value is high (e.g., >0.5), the observed value may be determined to belong to the seizure state (e.g., >0.5 to 1). If the observation value is low, the observed value may be determined to belong to the non-seizure state (e.g., ≤0.5). In various example embodiments, the statistical model may be configured to further generate a decision or output indication of a seizure state or a non-seizure state. For example, the statistical model may be configured to generate probability value (or statistical output) 0 indicating a non-seizure state and/or probability value (or statistical output) 1 indicating a seizure state. For example, the output indication by the statistical model may be statistical output 0 or statistical output 1, obtained by setting the observation or probability values to 1 if the probability is above a certain predetermined threshold, and setting the observation or probability values to 0 otherwise.

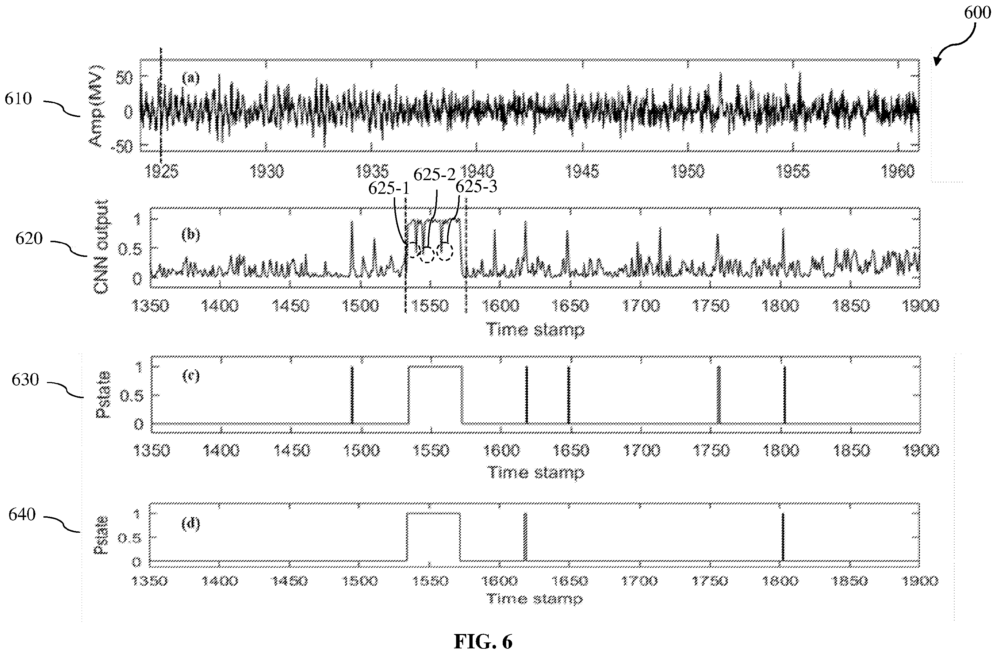

FIG. 6 illustrates an exemplary diagram 600 of brain signal data (e.g., comprising EEG signal segment 610), CNN classification output data (first processed output data) and post-processing output data (second processed output data) by the statistical models (e.g., HMM, CRF) on a test fold (e.g., in test data from patient 'CHB 15'). As illustrated, EEG signal segment 610 comprises a seizure event, where the onset is marked by a black dashed line. CNN output data (first processed output data) 620 for the same EEG signal segment 610 is illustrated, where the onset and offset are marked by black dashed lines. If a CNN output is greater than 0.5 the EEG segment may be determined or detected as a seizure; otherwise, it is detected as non-seizure EEG. As illustrated, there are outliers or instabilities, e.g., 625-1, 625-2, 625-3, in the CNN output data 620 during the seizure period, fluctuating rapidly in time. Second processed output data 630 using CNN and HMM of the EEG signal segment 610 and second processed output data 640 using CNN and CRF of the EEG signal segment 610 is shown. After post-processing using the statistical models, undesired outliers or fluctuations are removed by both statistical models. In addition, the FPs are reduced, and the false detection rate (FDR) is lowered.

In various example embodiments, as the spectral contents of the EEG signals are important for the detection of epileptic seizures, the detection method may be improved by segmenting the EEG signal into five separate spectral or frequency bands for delta (e.g., $\delta$: $1 \leq f \leq 4$Hz), theta (e.g., $\theta$: $4 \leq f \leq 8$Hz), alpha (e.g., $\alpha$: $8 \leq f \leq 13$Hz), beta (e.g., $\beta$: $13 \leq f \leq 30$ Hz), and gamma (e.g., $\gamma$: $\geq 30$ Hz). These sub-bands provide more accurate information about epileptic neuronal activities and consequently, some changes in the EEG signal, which are not so obvious in the original full-spectrum signal. The segmentation may enable rhythmic activity to be assessed or investigated. In addition, the changes in the neuronal activities may not spread out across the entire EEG spectrum, but are limited to certain frequency bands. Different combinations of sub-bands are fed into the CNN, either concatenated into one long vector, or arranged as a matrix. The CNN is then trained as usual. Next performance measures may be evaluated for various combinations of frequency bands.

In various example embodiments, a parallel and distributed deep learning framework may be implemented to reduce the training time. In various example embodiments, the deep CNN models may be designed in Python (v3.5.2) with TensorFlow 1.5.0 with a K80 Tesla GPU. For example, TensorFlow enables deep learning applications to be delivered easily into less powerful devices in a way that is not possible in the past. A pre-trained model may be used and then optimized to run on less-powerful offline devices such as PCs, laptops and mobile devices and still collect results in real-time.

The performance of the automated seizure detection framework was evaluated based on three different performance measures, (i) sensitivity, (ii) false detection rate (FDR), and (iii) latency. Sensitivity is the percentage of true seizure events detected by the system in the test dataset. FDR is calculated by the number of times the method misclassifies an EEG segment as seizure event per hour. Latency is the time delay (in sec) between the seizure detected by the method and the seizure onset marked by the clinical experts. Tables 2a-2c show performance of the seizure detection using for CNN, CNN and HMM, CNN and CRF, where average values are highlighted in bold, as follows:

Table 2a: Sensitivity (%) in each fold. TPs: true positives

| Fold No. | EEG duration (hh:mm) | No. of seizures | CNN | CNN+HMM | CNN+CRF |
|---|---|---|---|---|---|
| 1 | 350:20 | 37 | 94.59 | 97.30 | 94.59 |
| 2 | 155:86 | 62 | 87.10 | 88.71 | 95.16 |
| 3 | 235:36 | 29 | 75.86 | 79.31 | 86.21 |
| 4 | 210:50 | 53 | 88.68 | 94.34 | 90.57 |
| | **951:92** | **181** | **86.56** | **89.91** | **91.63** |

Table 2b: False detection rate (FDR) per hour in each fold. FPs: false positive

| Fold No. | CNN FPs | CNN FDR/h | CNN + HMM FPs | CNN + HMM FDR/h | CNN+CRF FPs | CNN + CRF FDR/h |
|---|---|---|---|---|---|---|
| 1 | 88 | 0.59 | 54 | 0.36 | 50 | 0.33 |
| 2 | 77 | 0.68 | 41 | 0.38 | 43 | 0.38 |
| 3 | 89 | 0.66 | 68 | 0.50 | 56 | 0.41 |
| 4 | 98 | 0.74 | 56 | 0.42 | 32 | 0.24 |
| | 352 | **0.67** | 219 | **0.41** | 181 | **0.34** |

Table 2c: Detection latency (in seconds) in each fold

| Fold No. | CNN | CNN+HMM | CNN+CRF |
|---|---|---|---|
| 1 | 1.74 | 4.85 | 3.45 |
| 2 | 1.98 | 4.80 | 4.37 |
| 3 | 2.20 | 4.75 | 3.95 |
| 4 | 2.47 | 4.65 | 2.85 |
| | **2.10** | **4.77** | **3.65** |

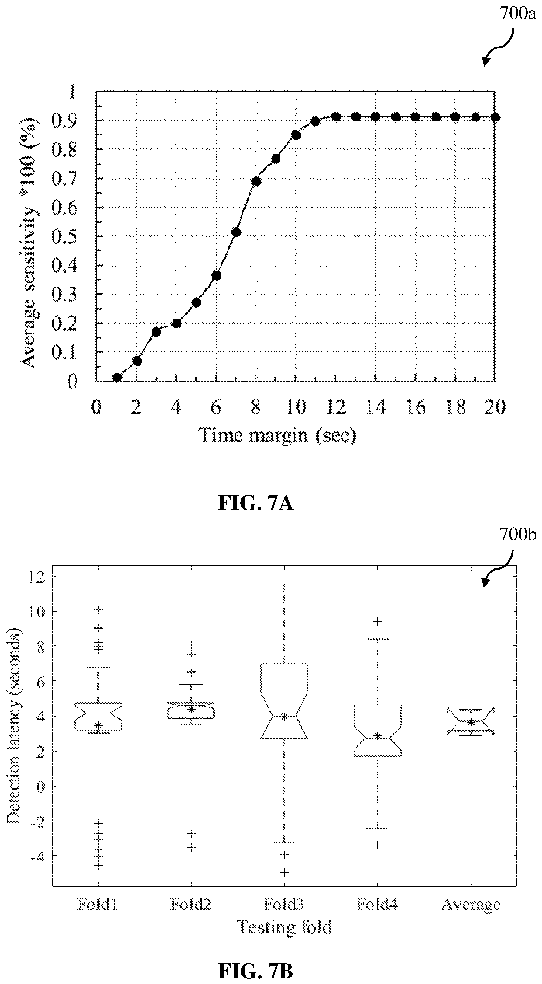

It can be observed that CNN and CRF yields the best performance: an average detection sensitivity of 91.63%, an average FDR of 0.34 per hour, and an average detection latency of 3.65 seconds. In various example embodiments, the CRF model may be advantageous over HMM. For example, the HMMs may not be capable of modeling complex interdependencies within the observed variables of the CNN output data. In various example embodiments, the CNN and CRF model may be able to detect 167 out of 181 seizure events, which corresponds to an average sensitivity of 91.63% (see Table 2a), across all the folds. The sensitivity for varying values of the time margin, i.e., the gap between the detected and actual onset may be calculated for the processed output data obtained by the CNN and CRF model. As shown in graph 700a in FIG. 7A, the sensitivity increases with the time margin, as expected. The sensitivity saturates at a time margin of 12 seconds.

Referring to Table 2b, it shows the number and rate of FPs for each testing fold. The average FDR for the CNN and CRF system is 0.34 per hour. The algorithm relies on signatures of seizure EEG recordings for detecting seizures. However, non-seizure EEG recordings may comprise similar signatures, which include unmarked sub-clinical seizures, rhythmic spike discharges, and high-amplitude artifacts.

In various example embodiments, the CNN and CRF algorithm attained an average latency of 3.65 seconds (see Table 2c) and average median latency of 3.70 seconds.

FIG. 7B shows the box plot 700b for detection latencies: average detection latency along with the maximum and minimum recoded latencies for each of the four test folds. A seizure event associated with 'Fold 3' achieved the minimum latency of -4.95 seconds. Negative latency indicates seizure event detection before visual identification on EEG recording. The latency also depends on the seizure onset characteristics in the training data. A slowly starting seizure with oscillatory characteristics that develop over time or a seizure onset buried in artifacts may easily induce a delay in detection. A seizure event of 'Fold 3' have the largest detection latency of 11.78 seconds.

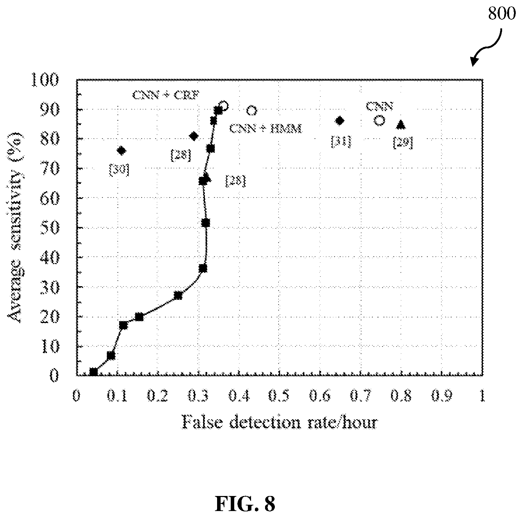

FIG. 8 shows an exemplary graph 800 illustrating the sensitivity vs. the FDR of the hybrid seizure detection framework according to various embodiments of the present invention along with the different studies proposed in the literature. The plot shows the performance of the hybrid seizure detection framework (black open circles), the results of different generalized seizure detectors evaluated on the CHB-MIT dataset in the literature (black triangles), and the various other generalized seizure detector results (black diamonds). Most studies in the literature did not consider the latency as a performance measure. In contrast, the CNN+CRF method considered latency performance and attained an average detection latency of 3.65 seconds and a median seizure detection latency of 3.70 seconds, which is lower than that achieved in Furbass F, Kampusch S, Kaniusas E, Koren J, Pirker S, Hopfengentner R, et al. Automatic multimodal detection for long-term seizure documentation in epilepsy. Clinical Neurophysiology. 2017;128(8):1466-1472.] (26 seconds). Dataset considered in Furbass F, P PO, Hartmann M, Perko H, Skupch AM, Lindinger G, et al. Prospective multi-center study of an automatic online seizure detection system for epilepsy monitoring units. Clinical Neurphysiology. 2015;126(6):1124-31, and Thodoroff P, Pineau J, Lim A. Learning Robust Features using Deep Learning for Automatic Seizure Detection. In: Proceedings of the 1st Machine Learning for Healthcare Conference. vol. 56, is the same as used in assessing the present framework, and hence the results reported in those papers can serve as direct benchmark.

As described, a hybrid machine learning system and method may be provided by combining a deep neural network and a statistical model for automatic seizure detection. In various example embodiments, the CNN+CRF based method has achieved best results with an average sensitivity of 91.63%, an average FDR of 0.34 per hour and an average detection latency of 3.65 seconds from long-term EEG scalp recordings. The achieved results reaches the state-of the art performance on generalized seizure detection performance. Automation of this process may be clinically useful in the long-term EEG monitoring practice and treatment planning for epilepsy patients. The system may be clinically useful in the review of long-term scalp EEG recordings for accurate diagnosis and identification of epileptic seizures as well as seizures resulting from other neurological causes. It may reduce the burden on the experts thereby enabling them to spend their time effectively in treatment planning for epilepsy patients.

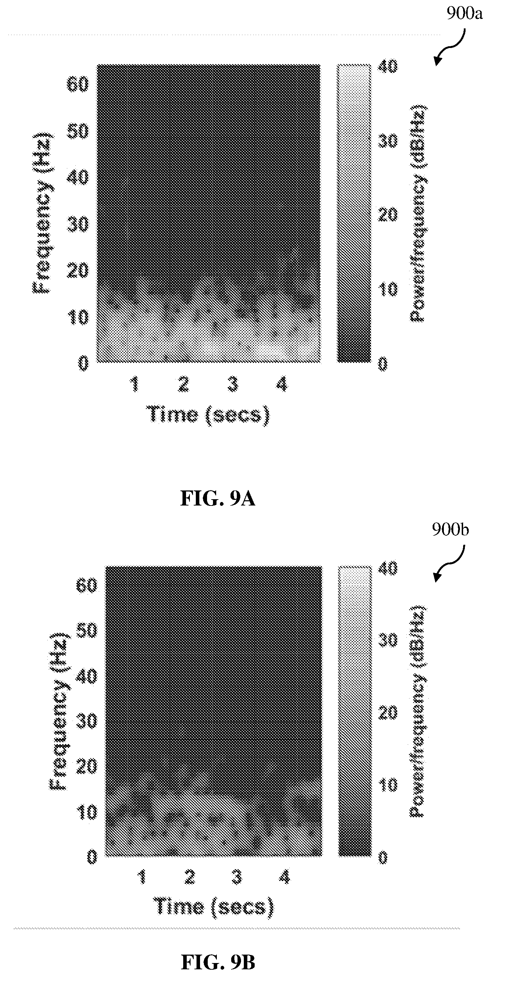

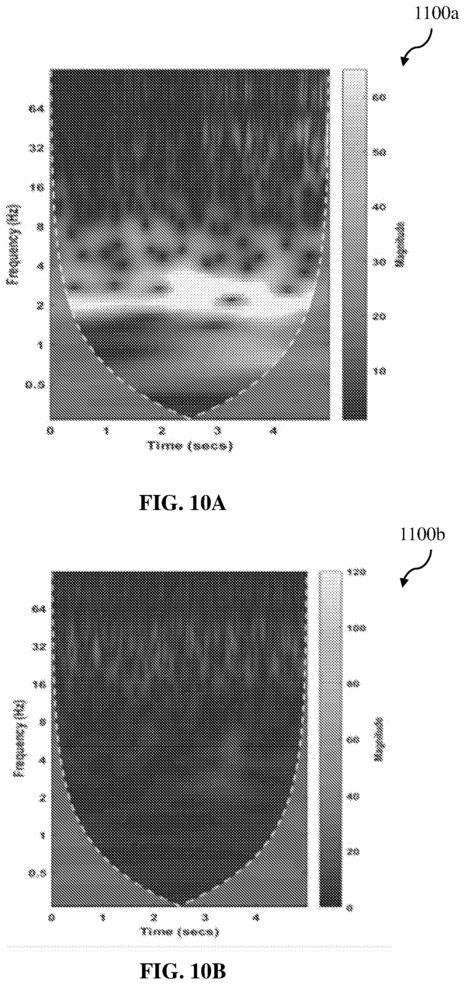

FIGS. 9A-9B illustrate exemplary STFT spectrograms 900a and 900b for the seizure EEG segments and non-seizure EEG segments, respectively (not illustrated with color scale). FIGS. 10A-10B illustrate exemplary scalograms 1000a and 1000b (e.g., continuous wavelet transform) with a Morlet wavelet basis for the seizure EEG segments and non-seizure EEG segments, respectively (not illustrated with color scale). A dashed line denotes the cone of influence. Within this region, the wavelet coefficient estimates may be reliable.

While embodiments of the invention have been particularly shown and described with reference to specific embodiments, it should be understood by those skilled in the art that various changes in form and detail may be made therein without departing from the scope of the invention as defined by the appended claims. The scope of the invention is thus indicated by the appended claims.

## Claims

1.    A computer-implemented method for seizure detection using at least one processor, the method comprising:

processing (102) pre-recorded multi-channel brain signal data using a deep neural network to obtain a first processed output data of the brain signal data, wherein the deep neural network is trained using a brain signal dataset comprising brain signal segments relating to seizure events and brain signal segments relating to non-seizure events, the first processed output data indicating one or more seizure events in the brain signal data and comprising outliers of the processed multi-channel brain signal data;

processing (106) the first processed output data from the deep neural network using a statistical model to obtain a second processed output data of the brain signal data, wherein the statistical model is configured to model transitions between the one or more seizure events and one or more non-seizure events in the first processed output data of the brain signal data and reduce the outliers of the processed multi-channel brain signal data; and

determining (108) the one or more seizure events based on the second processed output data of the brain signal data.

2.    The method of claim 1, wherein the brain signal data processed using the deep neural network comprises spectral, temporal and spatial information in relation to the one or more seizure events.

3.    The method of claim 1 or 2, further comprising producing an image-based representation of the brain signal data in the time-frequency domain, wherein said processing the brain signal data using a deep neural network comprises processing the image-based representation to obtain the first processed output data.

4.    The method of any one of claims 1 to 3, wherein the brain signal data comprises a plurality of signals, each of the plurality of signals corresponding to a respective channel that is associated with a different brain spatial location, and said processing the brain signal data using a deep neural network further comprises convolving each of the plurality of signals corresponding to a respective channel with a one-dimensional linear finite impulse response filter.

5. The method of any one of claims 1 to 4, further comprising segmenting the brain signal data into a plurality of different spectral bands.

6. The method of any one of claims 1 to 5, wherein the statistical model comprises a Hidden Markov Model (HMM) or a Conditional Random Field (CRF).

7. The method of any one of claims 1 to 6, wherein the brain signal data comprises electroencephalogram (EEG) signal data acquired using an EEG device.

8. A system (200) for seizure detection, the system (200) comprising:

a memory (204); and
at least one processor (206) communicatively coupled to the memory (204) and configured to:

obtain multi-channel brain signal data of brain electrical activity of a subject;
process the multi-channel brain signal data using a deep neural network to obtain a first processed output data of the brain signal data, wherein the deep neural network is trained using a brain signal dataset comprising brain signal segments relating to seizure events and brain signal segments relating to non-seizure events, the first processed output data indicating one or more seizure events in the brain signal data and comprising outliers of the processed multi-channel brain signal data;
process the first processed output data from the deep neural network using a statistical model to obtain a second processed output data of the brain signal data, wherein the statistical model is configured to model transitions between the one or more seizure events and one or more non-seizure events in the first processed output data of the brain signal data and reduce the outliers of the processed multi-channel brain signal data; and
determine the one or more seizure events based on the second processed output data of the brain signal data.

9. The system of claim 8, wherein the brain signal data processed using the deep neural network comprises spectral, temporal and spatial information in relation to the one or more seizure events.

10. The system of claim 8 or 9, further comprising producing an image-based representation of the brain signal data in the time-frequency domain, wherein said processing the brain signal data using a deep neural network comprises processing the image-based representation to obtain the first processed output data.

11. The system of any one of claims 8 to 10, wherein the brain signal data comprises a plurality of signals, each of the plurality of signals corresponding to a respective channel that is associated with a different brain spatial location, and said processing the brain signal data using a deep neural network further comprises convolving each of the plurality of signals corresponding to a respective channel with a one-dimensional linear finite impulse response filter.

12. The system of any one of claims 8 to 11, wherein the statistical model comprises a Hidden Markov Model (HMM) or a Conditional Random Field (CRF).

13. The system of any one of claims 8 to 12, further comprising segmenting the brain signal data into a plurality of different spectral bands.

14. The system of any one of claims 8 to 13, wherein the brain signal data comprises electroencephalogram (EEG) signal data acquired using an EEG device.

15. A computer program product, embodied in one or more non-transitory computer-readable storage mediums, comprising instructions executable by at least one processor (206) to perform a method for seizure detection, the method comprising:

obtaining multi-channel brain signal data of brain electrical activity of a subject;

**EP 3 920 781 B1**

processing the multi-channel brain signal data using a deep neural network to obtain a first processed output data of the brain signal data, wherein the deep neural network is trained using a brain signal dataset comprising brain signal segments relating to seizure events and brain signal segments relating to non-seizure events, the first processed output data indicating one or more seizure events in the brain signal data and comprising outliers of the processed multi-channel brain signal data;

processing the first processed output data from the deep neural network using a statistical model to obtain a second processed output data of the brain signal data, wherein the statistical model is configured to model transitions between the one or more seizure events and one or more non-seizure events in the first processed output data of the brain signal data and reduce the outliers of the processed multi-channel brain signal data; and

determining the one or more seizure events based on the second processed output data of the brain signal data.

**Patentansprüche**

1. Ein computerimplementiertes Verfahren zur Anfallserkennung unter Verwendung mindestens eines Prozessors, aufweisend:

   Verarbeiten (102) von zuvor aufgezeichneten mehrkanaligen Hirnsignaldaten mithilfe eines tiefen neuronalen Netzwerks, um erste verarbeitete Ausgabedaten der Hirnsignaldaten zu erhalten, wobei das tiefe neuronale Netzwerk mithilfe eines Hirnsignaldatensatzes trainiert ist, der Hirnsignalsegmente für Anfallsereignisse und Hirnsignalsegmente für Nicht-Anfallsereignisse aufweist, die ersten verarbeiteten Ausgabedaten auf ein oder mehrere Anfallsereignisse in den Hirnsignaldaten hinweisen und Ausreißer der verarbeiteten mehrkanaligen Hirnsignaldaten aufweisen.

   Verarbeiten (106) der ersten verarbeiteten Ausgabedaten des tiefen neuronalen Netzwerks mithilfe eines statistischen Modells, um zweite verarbeitete Ausgabedaten der Hirnsignaldaten zu erhalten, wobei das statistische Modell so konfiguriert ist, dass es Übergänge zwischen dem einen oder den mehreren Anfallsereignissen und einem oder mehreren Nicht-Anfallsereignissen in den ersten verarbeiteten Ausgabedaten der Hirnsignaldaten modelliert und die Ausreißer der verarbeiteten mehrkanaligen Hirnsignaldaten reduziert, und

   Bestimmen (108) des einen oder der mehreren Anfallsereignisse basierend auf den zweiten verarbeiteten Ausgabedaten der Gehirnsignaldaten.

2. Das Verfahren gemäß Anspruch 1, wobei die unter Verwendung des tiefen neuronalen Netzwerks verarbeiteten Gehirnsignaldaten spektrale, zeitliche und räumliche Informationen in Bezug auf das einen oder die mehreren Anfallsereignisse aufweisen.

3. Das Verfahren gemäß Anspruch 1 oder 2, das ferner das Erzeugen einer bildbasierten Darstellung der Gehirnsignaldaten im Zeit-Frequenz-Bereich aufweist, wobei das Verarbeiten der Gehirnsignaldaten unter Verwendung eines tiefen neuronalen Netzwerks das Verarbeiten der bildbasierten Darstellung aufweist, um die ersten verarbeiteten Ausgabedaten zu erhalten.

4. Das Verfahren gemäß einem der Ansprüche 1 bis 3, wobei die Gehirnsignaldaten eine Vielzahl von Signalen aufweisen, wobei jedes der Vielzahl von Signalen einem jeweiligen Kanal entspricht, der mit einer anderen räumlichen Position im Gehirn verknüpft ist, und wobei die Verarbeitung der Gehirnsignaldaten unter Verwendung eines tiefen neuronalen Netzwerks ferner das Falten jedes der Vielzahl von Signalen, die einem jeweiligen Kanal entsprechen, mit einem eindimensionalen linearen Filter mit endlicher Impulsantwort aufweist.

5. Das Verfahren gemäß einem der Ansprüche 1 bis 4, das ferner das Segmentieren der Gehirnsignaldaten in eine Vielzahl unterschiedlicher Spektralbänder aufweist.

6. Das Verfahren gemäß einem der Ansprüche 1 bis 5, wobei das statistische Modell ein Hidden-Markov-Modell (HMM) oder ein Conditional Random Field (CRF) aufweist.

7. Das Verfahren gemäß einem der Ansprüche 1 bis 6, wobei die Gehirnsignaldaten Elektroenzephalogramm-Signaldaten (EEG) aufweisen, die unter Verwendung eines EEG-Geräts erfasst wurden.

8. Eine Vorrichtung (200) zur Anfallserkennung, aufweisend:

   einen Speicher (204); und

15

mindestens einen Prozessor (206), der mit dem Speicher (204) kommunikativ verbunden ist und konfiguriert ist, um:

mehrkanalige Hirnsignaldaten der elektrischen Hirnaktivität eines Probanden zu erfassen;

die mehrkanaligen Hirnsignaldaten mithilfe eines tiefen neuronalen Netzwerks zu verarbeiten, um erste verarbeitete Ausgabedaten der Hirnsignaldaten zu erhalten, wobei das tiefe neuronale Netzwerk mithilfe eines Hirnsignaldatensatzes trainiert ist, der Hirnsignalsegmente im Zusammenhang mit Anfallsereignissen und Hirnsignalsegmente im Zusammenhang mit Nicht-Anfallsereignissen aufweist, wobei die ersten verarbeiteten Ausgabedaten auf ein oder mehrere Anfallsereignisse in den Hirnsignaldaten hinweisen und Ausreißer der verarbeiteten mehrkanaligen Hirnsignaldaten aufweisen;

die ersten verarbeiteten Ausgabedaten des tiefen neuronalen Netzwerks mithilfe eines statistischen Modells zu bearbeiten, um zweite verarbeitete Ausgabedaten der Gehirnsignaldaten zu erhalten, wobei das statistische Modell so konfiguriert ist, dass es Übergänge zwischen dem einen oder den mehreren Anfallsereignissen und einem oder mehreren Nicht-Anfallsereignissen in den ersten verarbeiteten Ausgabedaten der Gehirnsignaldaten modelliert und die Ausreißer der verarbeiteten mehrkanaligen Gehirnsignaldaten reduziert; und

das eine oder die mehreren Anfallsereignisse basierend auf den zweiten verarbeiteten Ausgabedaten der Gehirnsignaldaten zu bestimmen.

9. Die Vorrichtung gemäß Anspruch 8, wobei die unter Verwendung des tiefen neuronalen Netzwerks verarbeiteten Gehirnsignaldaten spektrale, zeitliche und räumliche Informationen in Bezug auf das eine oder die mehreren Anfallsereignisse aufweisen.

10. Die Vorrichtung gemäß Anspruch 8 oder 9, die ferner das Erstellen einer bildbasierten Darstellung der Gehirnsignaldaten im Zeit-Frequenz-Bereich aufweist, wobei die Verarbeitung der Gehirnsignaldaten unter Verwendung eines tiefen neuronalen Netzwerks das Verarbeiten der bildbasierten Darstellung aufweist, um die ersten verarbeiteten Ausgabedaten zu erhalten.

11. Die Vorrichtung gemäß einem der Ansprüche 8 bis 10, wobei die Gehirnsignaldaten eine Vielzahl von Signalen umfassen, wobei jedes der Vielzahl von Signalen einem jeweiligen Kanal entspricht, der mit einer anderen räumlichen Position im Gehirn verknüpft ist, und wobei die Verarbeitung der Gehirnsignaldaten unter Verwendung eines tiefen neuronalen Netzwerks ferner das Falten jedes der Vielzahl von Signalen, die einem jeweiligen Kanal entsprechen, mit einem eindimensionalen linearen Filter mit endlicher Impulsantwort aufweist.

12. Die Vorrichtung gemäß einem der Ansprüche 8 bis 11, wobei das statistische Modell ein Hidden-Markov-Modell (HMM) oder ein Conditional Random Field (CRF) aufweist.

13. Die Vorrichtung gemäß einem der Ansprüche 8 bis 12, das ferner das Segmentieren der Gehirnsignaldaten in eine Vielzahl unterschiedlicher Spektralbänder aufweist.

14. Die Vorrichtung gemäß einem der Ansprüche 8 bis 13, wobei die Gehirnsignaldaten Elektroenzephalogramm-Signaldaten (EEG) aufweisen, die unter Verwendung eines EEG-Geräts erfasst wurden.

15. Ein Computerprogrammprodukt, das auf einem oder mehreren nichtflüchtigen computerlesbaren Speichermedien gespeichert ist und Anweisungen enthält, die von mindestens einem Prozessor (206) ausgeführt werden können, um ein Verfahren zur Anfallserkennung durchzuführen, aufweisend:

Erfassen mehrkanaliger Hirnsignaldaten der elektrischen Hirnaktivität eines Probanden;

Verarbeiten der mehrkanaligen Hirnsignaldaten mithilfe eines tiefen neuronalen Netzwerks, um erste verarbeitete Ausgabedaten der Hirnsignaldaten zu erhalten, wobei das tiefe neuronale Netzwerk mithilfe eines Hirnsignaldatensatzes trainiert ist, der Hirnsignalsegmente mit Bezug zu Anfallsereignissen und Hirnsignalsegmente mit Bezug zu Nicht-Anfallsereignissen aufweist, die ersten verarbeiteten Ausgabedaten auf ein oder mehrere Anfallsereignisse in den Hirnsignaldaten hinweisen und Ausreißer der verarbeiteten mehrkanaligen Hirnsignaldaten aufweisen.

Verarbeiten der ersten verarbeiteten Ausgabedaten des tiefen neuronalen Netzwerks mithilfe eines statistischen Modells, um zweite verarbeitete Ausgabedaten der Gehirnsignaldaten zu erhalten, wobei das statistische Modell so konfiguriert ist, dass es Übergänge zwischen dem einen oder den mehreren Anfallsereignissen und einem oder mehreren Nicht-Anfallsereignissen in den ersten verarbeiteten Ausgabedaten der Gehirnsignaldaten modelliert und die Ausreißer der verarbeiteten mehrkanaligen Gehirnsignaldaten reduziert; und

Bestimmen des einen oder der mehreren Anfallsereignisse basierend auf den zweiten verarbeiteten Ausgabedaten der Gehirnsignaldaten.

**Revendications**

1. Procédé mis en œuvre par ordinateur pour une détection de crise d'épilepsie à l'aide d'au moins un processeur, le procédé comprenant :

   le traitement (102) de données de signaux cérébraux multicanaux préenregistrés à l'aide d'un réseau neuronal profond pour obtenir de premières données de sortie traitées des données de signaux cérébraux, dans lequel le réseau neuronal profond est entraîné à l'aide d'un ensemble de données de signaux cérébraux comprenant des segments de signaux cérébraux relatifs à des événements de crise d'épilepsie et des segments de signaux cérébraux relatifs à des événements autres qu'une crise d'épilepsie, les premières données de sortie traitées indiquant un ou plusieurs événements de crise d'épilepsie dans les données de signaux cérébraux et comprenant des valeurs aberrantes des données de signaux cérébraux multicanaux traitées ;
   le traitement (106) des premières données de sortie traitées provenant du réseau neuronal profond à l'aide d'un modèle statistique pour obtenir de secondes données de sortie traitées des données de signaux cérébraux, dans lequel le modèle statistique est configuré pour modéliser des transitions entre les un ou plusieurs événements de crise d'épilepsie et un ou plusieurs événements autres qu'une crise d'épilepsie dans les premières données de sortie traitées des données de signaux cérébraux et réduire les valeurs aberrantes des données de signaux cérébraux multicanaux traitées ; et
   la détermination (108) des un ou plusieurs événements de crise d'épilepsie sur la base des secondes données de sortie traitées des données de signaux cérébraux.

2. Procédé selon la revendication 1, dans lequel les données de signaux cérébraux traitées en utilisant le réseau neuronal profond comprennent des informations spectrales, temporelles et spatiales en relation avec les un ou plusieurs événements de crise d'épilepsie.

3. Procédé selon la revendication 1 ou 2, comprenant en outre la production d'une représentation basée sur une image des données de signaux cérébraux dans le domaine temps-fréquence, dans lequel ledit traitement des données de signaux cérébraux en utilisant un réseau neuronal profond comprend le traitement de la représentation basée sur une image pour obtenir les premières données de sortie traitées.

4. Procédé selon l'une quelconque des revendications 1 à 3, dans lequel les données de signaux cérébraux comprennent une pluralité de signaux, chaque signal de la pluralité de signaux correspondant à un canal respectif qui est associé à un emplacement spatial cérébral différent, et ledit traitement des données de signaux cérébraux en utilisant un réseau neuronal profond comprend en outre la convolution de chaque signal de la pluralité de signaux correspondant à un canal respectif avec un filtre linéaire unidimensionnel à réponse impulsionnelle finie.

5. Procédé selon l'une quelconque des revendications 1 à 4, comprenant en outre la segmentation des données de signaux cérébraux en une pluralité de bandes spectrales différentes.

6. Procédé selon l'une quelconque des revendications 1 à 5, dans lequel le modèle statistique comprend un modèle de Markov caché (HMM) ou un champ aléatoire conditionnel (CRF).

7. Procédé selon l'une quelconque des revendications 1 à 6, dans lequel les données de signaux cérébraux comprennent des données de signaux d'électroencéphalogramme (EEG) acquises en utilisant un dispositif EEG.

8. Système (200) pour une détection de crise d'épilepsie, le système (200) comprenant : une mémoire (204) ; et au moins un processeur (206) couplé en communication à la mémoire (204) et configuré pour :

   obtenir des données de signaux cérébraux multicanaux de l'activité électrique cérébrale d'un sujet ;
   traiter les données de signaux cérébraux multicanaux à l'aide d'un réseau neuronal profond pour obtenir de premières données de sortie traitées des données de signaux cérébraux, dans lequel le réseau neuronal profond est entraîné à l'aide d'un ensemble de données de signaux cérébraux comprenant des segments de signaux cérébraux relatifs à des événements de crise d'épilepsie et des segments de signaux cérébraux relatifs à des événements autres qu'une crise d'épilepsie, les premières données de sortie traitées indiquant un ou plusieurs événements de crise d'épilepsie dans les données de signaux cérébraux et comprenant des valeurs aberrantes des données de signaux cérébraux multicanaux traitées ;

traiter les premières données de sortie traitées provenant du réseau neuronal profond à l'aide d'un modèle statistique pour obtenir de secondes données de sortie traitées des données de signaux cérébraux, dans lequel le modèle statistique est configuré pour modéliser des transitions entre les un ou plusieurs événements de crise d'épilepsie et un ou plusieurs événements autres qu'une crise d'épilepsie dans les premières données de sortie traitées des données de signaux cérébraux et réduire les valeurs aberrantes des données de signaux cérébraux multicanaux traitées ; et

déterminer les un ou plusieurs événements de crise d'épilepsie sur la base des secondes données de sortie traitées des données de signaux cérébraux.

9. Système selon la revendication 8, dans lequel les données de signaux cérébraux traitées en utilisant le réseau neuronal profond comprennent des informations spectrales, temporelles et spatiales en relation avec les un ou plusieurs événements de crise d'épilepsie.

10. Système selon la revendication 8 ou 9, comprenant en outre la production d'une représentation basée sur une image des données de signaux cérébraux dans le domaine temps-fréquence, dans lequel ledit traitement des données de signaux cérébraux en utilisant un réseau neuronal profond comprend le traitement de la représentation basée sur une image pour obtenir les premières données de sortie traitées.

11. Système selon l'une quelconque des revendications 8 à 10, dans lequel les données de signaux cérébraux comprennent une pluralité de signaux, chaque signal de la pluralité de signaux correspondant à un canal respectif qui est associé à un emplacement spatial cérébral différent, et ledit traitement des données de signaux cérébraux en utilisant un réseau neuronal profond comprend en outre la convolution de chaque signal de la pluralité de signaux correspondant à un canal respectif avec un filtre linéaire unidimensionnel à réponse impulsionnelle finie.

12. Système selon l'une quelconque des revendications 8 à 11, dans lequel le modèle statistique comprend un modèle de Markov caché (HMM) ou un champ aléatoire conditionnel (CRF).

13. Système selon l'une quelconque des revendications 8 à 12, comprenant en outre la segmentation des données de signaux cérébraux en une pluralité de bandes spectrales différentes.

14. Système selon l'une quelconque des revendications 8 à 13, dans lequel les données de signaux cérébraux comprennent des données de signaux d'électroencéphalogramme (EEG) acquises en utilisant un dispositif EEG.

15. Produit programme d'ordinateur, incorporé dans un ou plusieurs supports de stockage non transitoires lisibles par ordinateur, comprenant des instructions exécutables par au moins un processeur (206) pour mettre en œuvre un procédé de détection de crise d'épilepsie, le procédé comprenant :

l'obtention de données de signaux cérébraux multicanaux de l'activité électrique cérébrale d'un sujet ;

le traitement des données de signaux cérébraux multicanaux à l'aide d'un réseau neuronal profond pour obtenir de premières données de sortie traitées des données de signaux cérébraux, dans lequel le réseau neuronal profond est entraîné à l'aide d'un ensemble de données de signaux cérébraux comprenant des segments de signaux cérébraux relatifs à des événements de crise d'épilepsie et des segments de signaux cérébraux relatifs à des événements autres qu'une crise d'épilepsie, les premières données de sortie traitées indiquant un ou plusieurs événements de crise d'épilepsie dans les données de signaux cérébraux et comprenant des valeurs aberrantes des données de signaux cérébraux multicanaux traitées ;

le traitement des premières données de sortie traitées provenant du réseau neuronal profond à l'aide d'un modèle statistique pour obtenir de secondes données de sortie traitées des données de signaux cérébraux, dans lequel le modèle statistique est configuré pour modéliser des transitions entre les un ou plusieurs événements de crise d'épilepsie et un ou plusieurs événements autres qu'une crise d'épilepsie dans les premières données de sortie traitées des données de signaux cérébraux et réduire les valeurs aberrantes des données de signaux cérébraux multicanaux traitées ; et

la détermination des un ou plusieurs événements de crise d'épilepsie sur la base des secondes données de sortie traitées des données de signaux cérébraux.

100

| obtaining brain signal data of brain electrical activity of a subject | 102 |

| processing the brain signal data using a deep neural network to obtain a first processed output data of the brain signal data, the first processed output data indicating one or more seizure events in the brain signal data | 104 |

| processing the first processed output data using a statistical model to obtain a second processed output data of the brain signal data, wherein the statistical model is configured to model transitions between the one or more seizure events and one or more non-seizure events in the first processed output data of the brain signal data | 106 |

| determining the one or more seizure events based on the second processed output data of the brain signal data | 108 |

**FIG. 1**

200

Processor

Memory

206

204

Brain Signal Data
Module

First Signal Processing
Module

Second Signal
Processing Module

Seizure Event
Determining Module

208

210

212

214

**FIG. 2**

**FIG. 3**

FIG. 4

FIG. 5A

510

**FIG. 5B**

520

**FIG. 5C**

**FIG. 6**

EP 3 920 781 B1

700a

**FIG. 7A**

700b

**FIG. 7B**

FIG. 8

**FIG. 9A**

**FIG. 9B**

1100a

**FIG. 10A**

1100b

**FIG. 10B**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- SG 10201901109 U

- US 2013274625 A1

**Non-patent literature cited in the description**

- **FURBASS F** ; **KAMPUSCH S** ; **KANIUSAS E** ; **KOREN J** ; **PIRKER S** ; **HOPFENGENTNER R et al.** Automatic multimodal detection for long-term seizure documentation in epilepsy.. *Clinical Neurophysiology.*, 2017, vol. 128 (8), 1466-1472

- **FURBASS F** ; **P PO** ; **HARTMANN M** ; **PERKO H** ; **SKUPCH AM** ; **LINDINGER G et al.** Prospective multi-center study of an automatic online seizure detection system for epilepsy monitoring units. *Clinical Neurphysiology.*, 2015, vol. 126 (6), 1124-31

- **THODOROFF P** ; **PINEAU J** ; **LIM A**. Learning Robust Features using Deep Learning for Automatic Seizure Detection. *Proceedings of the 1st Machine Learning for Healthcare Conference*, vol. 56